# EUROPEAN PATENT SPECIFICATION

(11) **EP 1 374 784 B1**
(45) Date of publication and mention of the grant of the patent: **30.07.2008**
(21) Application number: 03291442.6
(22) Date of filing: 16.06.2003
(51) Int. Cl.: A61B 17/17

(54) **Variable depth bone drill guide**
Tiefeneinstellbare Knochenbohrlehre
Guide pour percer un os avec profondeur variable

(30) Priority: 18.06.2002 US 174118
(43) Date of publication of application: 02.01.2004
(73) Proprietor: Stryker Spine, 33610 Cestas (FR)
(72) Inventor: Markworth, Aaron, Pompton Lakes, NJ 07442 (US)
(74) Representative: Le Forestier, Eric

(56) References cited:
- WO-A-01/28437
- WO-A-02/067786
- DE-A- 3 622 676
- US-A- 5 409 493
- US-A- 5 810 828

## Description

### Technical Field

This invention generally relates to surgical devices and more specifically relates to drill guides for use in drilling bones.

### Background Art

The spinal column is a highly complex system of bones and connective tissues that provides support for the body and protects the delicate spinal cord and nerves. The spinal column includes a series of vertebral bodies stacked one atop the other, each vertebral body including an inner or central portion of relatively weak cancellous bone and an outer portion of relatively strong cortical bone. Situated between each vertebral body is an intervertebral disc that cushions and dampens compressive forces exerted upon the spinal column. A vertebral canal containing the spinal cord and nerves is located behind the vertebral bodies.

There are many types of spinal column disorders, including scoliosis (abnormal lateral curvature of the spine), kyphosis (abnormal forward curvature of the spine, usually in the thoracic spine), excess lordosis (abnormal backward curvature of the spine, usually in the lumbar spine), spondylolisthesis (forward displacement of one vertebra over another, usually in a lumbar or cervical spine) and other disorders caused by abnormalities, disease or trauma, such as ruptured or slipped discs, degenerative disc disease, fractured vertebra, and the like. Patients that suffer from such conditions usually experience extreme and debilitating pain, as well as diminished nerve function.

Surgical techniques commonly referred to as spinal fixation use surgical implants and/or mechanical immobilization to fuse two or more vertebral bodies of the spinal column. Spinal fixation may also be used to alter the alignment of adjacent vertebral bodies relative to one another so as to change the overall alignment of the spinal column. Such techniques have been used effectively to treat the above-described conditions and, in many cases, to relieve pain.

One spinal fixation technique involves immobilizing the spine using orthopedic stabilizing rods, commonly referred to as spine rods, which are positioned generally parallel to the spine. This may be accomplished by exposing the spine posteriorly and fastening bone screws to the pedicles of vertebral bodies. The pedicle screws are generally placed two per vertebra and serve as anchor points for the spine rods.

In order to fasten pedicle bone screws, a hole must be drilled and, preferably tapped into the pedicles. It is important in orthopedic applications, such as inserting bone screws into the pedicles of vertebral bodies, that the depth of the hole being drilled is monitored. For example, if a hole is drilled or tapped into a bone too deeply, major damage may be caused to arteries or tissue surrounding the bone. The depth of a hole for receiving a pedicle screw is in the range of about 6mm to about 54mm and pedicle screws are readily available in these sizes. Preferably, the depth of the hole is about 10-14mm to receive pedicle screws of the same length. Moreover, it is equally as important to monitor the depth of tapping of the hole, since the flutes on the tap end can cause severe damage to arteries or other tissue surrounding the bone. Typically the depth of tapping a hole ranges from about 6mm to about 24mm. The preferred tapping depth depends on the depth of the hole and the length of the bone screw to be used.

Attempts have been made in the prior art to provide a guide for monitoring the depth of holes drilled into pedicles and other bones. Typically, a device usually referred to as a drill guide is utilized in connection with an orthopedic drill to gauge the depth of the hole. Such devices usually include a threaded collet and threaded slide, thus requiring the screwing on and off of the slide. The slide is screwed to a point on the collet that corresponds to the desired depth of the hole. A drill is then used in conjunction with the guide, so that the slide prevents the drill from drilling a hole past the desired depth.

For example, Glossop, U.S. Patent No. 6,203,543 B1 discloses a device for securing objects to bones using bone screws. Glossop recites drilling a hole prior to insertion of the bone screw. The device taught in Glossop is used to insert the bone screw into the hole. Glossop recites using a threaded nut on a sleeve. The screw is inserted axially in the sleeve. The threaded nut is set to prevent rotation of the screw into the bone beyond the desired depth.

The prior art systems such as the Glossop device, utilizing threaded members, are difficult to operate, since two hands are required to thread the slide to the proper depth. Since the drill guides are used in conjunction with drills, only one hand may be available to operate the guide. Additionally, a typical threaded slide does not lock into position, and therefore the depth may change. A simple drill guide is therefore needed which locks in at the desired depth. Moreover, the drill guide should be operable with only one hand.

Gisin et al., U.S. Patent No. 5,507,801 discloses the visual monitoring of the hole depth in a compression drill guide for drilling screw holes in bone fragments. The guide includes an outer cylinder, an inner cylinder with a greater length than the inner cylinder, and a spring mechanism for advancing the inner cylinder. The inner cylinder is provided with optical markings so that a surgeon can visually control the depth of the drilling. Thus, when the surgeon observes that the inner cylinder has reached the appropriate marking, the surgeon ceases drilling. Visual monitoring of the drilling of a hole in a bone is likely to result in errors in the hole being drilled too deeply or not deeply enough.

Kuslich et al., U.S. Patent No. 5,899,908 discloses a drill tube guide for use in spinal applications. A drill tube sleeve is utilized so that a bore is reamed until a shoulder of the reamer abuts the sleeve. The kit taught in Kuslich, however, includes multiple drill tubes, drill tube sleeves and reamers in various lengths to provide an accurate depth of the bore, to correspond to the size of the implant. Thus, the kit requires multiple drill guides to monitor different depths of the hole, providing a complex system.

The international publication No. WO 01/28437 discloses an adjustable guide tube having a distal end and a proximal end and includes guide housing having an adjustable sleeve that moves relative to the guide housing for adjusting the length of the adjustable guide tube between the distal end and the proximal end. A helical spring biases the adjustable sleeve and the guide housing away from one another. The guide housing has a series of slots that receive a pin on the adjustable sleeve. The pin interdigitates with slots to fix the overall distance between the rim of the adjustable sleeve and the contact edge of the guide housing.

Therefore, a guide is needed for monitoring the depth of a hole to be drilled in a bone that is simple to use and provides accurate results, so as to not cause damage to arteries, nerves and tissue surrounding the bone.

Additionally, a drill guide is needed that may be operated using only one hand, providing a surgeon with a free hand for operating the drill.

There is also a need for a drill guide that is adaptable to monitor the depth of a hole to various values.

In addition, there is a need for a guide that may be utilized to monitor the depth of tapping as well as drilling.

There is also a need for a drill guide that provides safety stops and locks to prevent the drill from drilling too deeply.

### Summary of the Invention

A drill guide is provided that overcomes the disadvantages of the prior art.

In accordance with the present invention, an instrument for use in drilling a bone as defined in the appended set of claims is provided. The instrument includes a sleeve with a depth gauge for setting the depth of a hole to be drilled into the bone. The sleeve defines an end for contacting said bone. The instrument further includes a slide having a releasable locking mechanism cooperable with the depth gauge. The slide is axially disposed within the sleeve. Additionally, the instrument includes a drill having a shaft defining a shoulder. The drill is axially received within the slide and advances to drill a hole in the bone until the shoulder abuts the slide.

Preferably, the depth gauge comprises a channel formed in the sleeve. The channel includes a plurality of pairs of notches. In addition, the locking mechanism is a spring arm formed in the slide. The arm includes an edge extending radially from its free end. The arm is adaptable to lock into a pair of notches, thereby locking the slide in position. The arm may be deflected toward the center of the slide to unlock the edge from the notches.

Preferably, the arm includes a tab extending radially from the arm and being aligned within the channel of the sleeve. Thus, a force may be applied to the tab to deflect the arm. Additionally, each of the pairs of notches may be labeled to identify the depth to which the pair corresponds.

In a preferred embodiment, the sleeve includes teeth extending axially therefrom for contacting the bone. The teeth contact the bone and ensure that the instrument is properly positioned. Moreover, the teeth prevent the instrument and the drill from slipping out of position.

According to another aspect of the invention, the drill defines a first end, a middle portion having a diameter smaller than the inner diameter of the slide and a second end. The shoulder is formed between the first end and the middle portion. Moreover, the middle portion fits axially within the slide.

In a preferred embodiment, the sleeve is comprised of stainless steel or titanium and the slide is comprised of stainless steel or titanium. Additionally, the slide may be formed via plastic injection molding. The depth gauge is capable of controlling the depth of the hole. In one embodiment of the invention used for the cervical spine, the depth gauge may be set to a depth of about 6mm to about 54mm, preferably between about 10mm and 14mm. Of course, the present invention may be utilized for other areas and therefore, the depth gauge may be set to various appropriate ranges.

According to another aspect of the invention, the instrument further includes a tap including a shaft defining a first shoulder. The tap may be axially inserted into the slide and advances to tap the hole in the bone until the first shoulder of the tap contacts or abuts the slide. In one embodiment of the invention used for the cervical spine, the depth gauge can control the depth of tapping in the range of about 6mm to about 24mm. Of course, the present invention contemplates applications other than the cervical spine and therefore the depth gauge for tapping may be set to other appropriate ranges according to the application for which it is used.

Additionally, the tap further defines a first end, a first middle portion having a diameter smaller than the diameter of the first end, and a second middle portion having a diameter smaller than the first middle portion. The first shoulder of the tap is formed between the first middle portion and the second middle portion and a second shoulder is formed between the first end and the first middle portion. The first end has a diameter larger than the inner diameter of the sleeve such that the second shoulder contacts or abuts the sleeve when the hole is tapped to a depth of about 24mm.

According to yet another aspect of the invention, an instrument for use in drilling a hole in a bone is provided comprising a sleeve, a slide, and a drill. The sleeve defines a channel including a plurality of pairs of notches. Each of the pairs of notches corresponds to a depth of the hole to be drilled into the bone. The sleeve further includes teeth extending axially therefrom for contacting the bone and preventing the instrument and drill from slipping from its position on the bone. The slide defines a spring arm having an edge extending radially from its free end. The edge, thus, locks into each of the pairs of notches in order to lock the slide in position. The arm may be deflected toward the center of the slide to unlock the edge from the notches and allow the slide to axially slide within the sleeve. The drill includes a shaft defining a shoulder. The shaft is axially inserted into the slide and advances to drill the hole in the bone until the shoulder contacts the slide. Since the depth of the hole has been set by lining the edge of the arm up with the pair of notches that corresponds to the desired depth, the hole will reach the desired depth when the shoulder abuts the slide.

Preferably the arm further includes a tab extending radially from the arm. The tab is aligned within the channel of the sleeve. Applying force to the tab deflects the arm, thereby unlocking the edge and allowing for axial movement of the slide. In a preferred embodiment, each of the pairs of notches is labeled with the corresponding depth.

According to another aspect of the invention, the drill defines a first end, a middle portion having a diameter smaller than the inner diameter of the slide and a second end, wherein the shoulder is formed between the first end and the middle portion.

In a preferred embodiment, the sleeve is comprised of stainless steel or titanium and the slide is comprised of stainless steel or titanium. Alternatively, the slide may be formed by plastic injection molding.

Preferably, when used for the cervical spine, the depth of the hole may be set from about 6mm to about 54mm.

In accordance with another aspect of the invention, the instrument further comprises a tap including a shaft defining a first shoulder. The tap is axially inserted into the slide and advances to tap the hole previously formed in the bone until the first shoulder of the tap contacts or abuts the slide. Preferably, when used for the cervical spine, the depth gauge is adaptable to control the depth of tapping in the range of about 6mm to about 24mm. According to a preferred embodiment, the tap defines a first end, a first middle portion having a diameter smaller than the diameter of the first end, and a second middle portion having a diameter smaller than the first middle portion. The first shoulder is formed between the first middle portion and the second middle portion and a second shoulder is formed between the first end and the first middle portion. Additionally, the first end has a diameter larger than the inner diameter of the sleeve so that the second shoulder contacts or abuts the sleeve when the hole is tapped to a depth of about 24mm.

According to another aspect not forming part of the invention, a method of drilling a hole in a bone using an instrument including a sleeve defining a channel including a plurality of pairs of notches and including teeth extending axially therefrom for contacting the bone, a slide defining a spring arm having an edge extending radially therefrom, the slide being axially disposed within the sleeve, and a drill including a shaft defining a shoulder is provided. The method includes creating a dimple on the surface of the bone at the location of the hole, setting the desired depth of the hole on the instrument, pressing the teeth to the bone to surround the dimple, inserting the shaft into the slide, drilling a hole in the bone with the shaft, and abutting the shoulder of the shaft on the slide.

Preferably the depth of the hole is set by applying a pressure to the arm to deflect the arm toward the center of the slide, axially moving the slide to line the edge up with the pair of notches that corresponds to the desired depth of the hole, and removing the pressure from the arm thereby locking the edge into the notches. The desired depth of the hole may be set to a depth of about 6mm to about 54mm when the present invention is used on the cervical spine.

In addition, another aspect not forming part of the invention contemplates tapping the hole, so that the hole can receive a threaded implant such as a bone screw. Thus, the method further includes removing the drill from the bone, removing the instrument from the bone, providing a tap defining a first shoulder and a second shoulder, setting the depth of the desired tapping on the instrument, pressing the teeth to the bone surrounding the hole, inserting the tap into the slide, tapping the hole with the tap, and abutting the second shoulder of the tap with the slide.

Preferably, the depth is set for tapping by applying a pressure to the arm to deflect the arm toward the center of the slide, axially moving the slide to line the edge up with the pair of notches that corresponds to the desired depth of the hole, and removing the pressure from the arm thereby locking the edge into the notches. The depth of the tapping may be set to a depth of about 6mm to about 24mm when the present invention is used for the cervical spine.

The maximum depth of the tapping is about 24mm when applied to the cervical spine. Of course, the maximum depth of tapping may be increased or decreased depending on the application of the tap. When the depth is set on the instrument to 24mm, the drill advances until the first shoulder of the tap contacts or abuts the sleeve.

### Brief Description of the Drawings

FIG. 1 is a perspective view of a drill guide having a drill shaft inserted in accordance with the present invention.
FIG. 2 is a perspective view of a sleeve in accordance with the present invention.
FIG. 3 is a perspective view of a slide in accordance with the present invention.
FIG. 4 is a sectional perspective view of the slide and sleeve in accordance with the present invention.
FIG. 5 is a sectional view of the slide and sleeve having a drill shaft inserted in accordance with the present invention.
FIG. 6 is a side view of a drill shaft in accordance with the present invention.
FIG. 7 is a side view of a tap in accordance with the present invention.

### Best Mode of Carrying Out The Invention

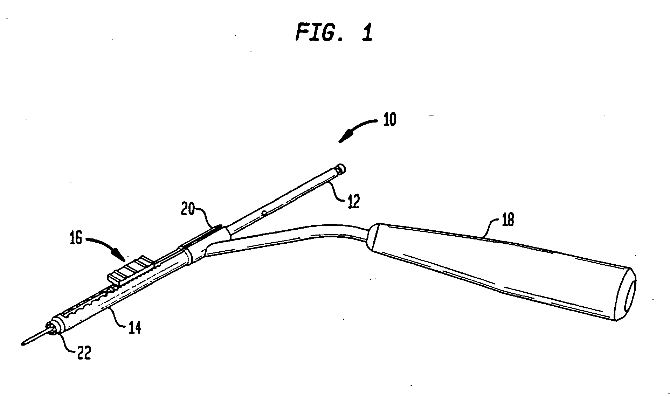

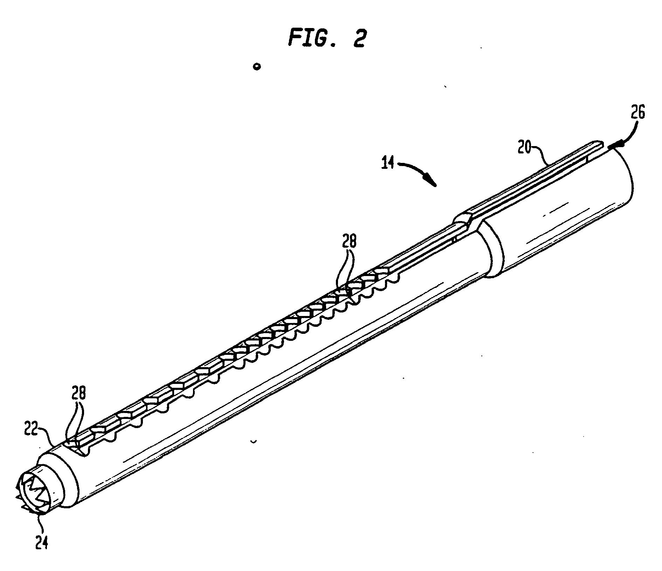

Referring to FIGS. 1-3, a drill guide 10 for use with an orthopedic drill shaft 12 or tap is shown. The guide 10 generally comprises a sleeve 14, a slide 16 and a handle 18.

The sleeve 14 is generally a hollow cylinder defining a proximal end 20 and a distal end 22. Serrations or teeth 24 extend axially from the distal end 22. The sleeve 14 further defines a channel 26 extending from the proximal end 20 and terminating at a point prior to the edge of the distal end 22.

The channel includes receiving members along its opposite edges. As illustrated herein, the receiving members comprise pairs of notches 28 disposed along the opposite edges of the channel. Of course, the present invention contemplates the receiving members being various shapes including a plurality of holes, indents, notches or other structures in the sleeve to engage the tab and prevent movement of the slide.

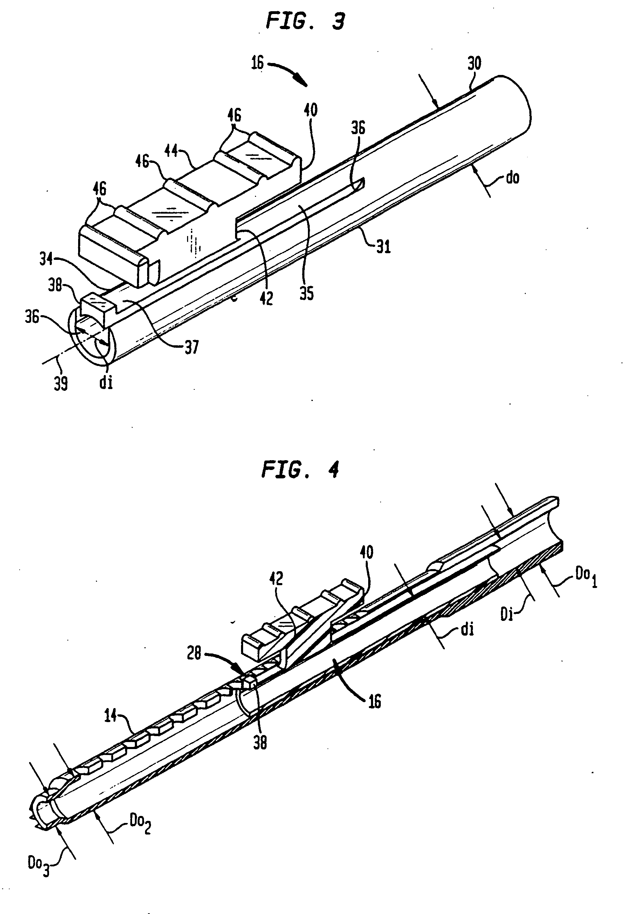

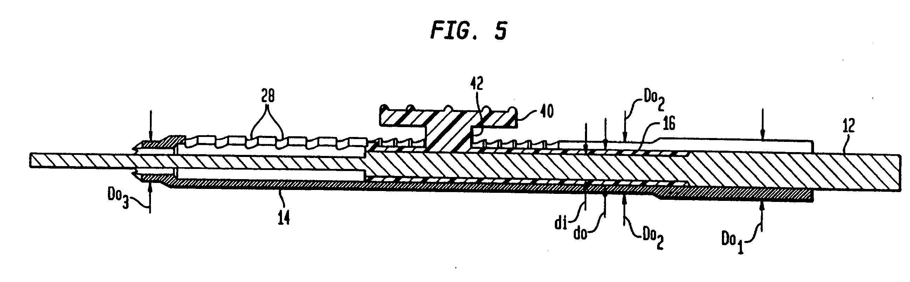

Each pair of notches 28 corresponds to a desired depth of the hole. Preferably, each pair of notches is labeled with its corresponding depth. The interior diameter Dᵢ of the sleeve remains constant, as shown in FIGS. 4 and 5. The outside diameter Do of the sleeve is stepped, having the greatest diameter Dₒ₁ at the proximal end 20, decreasing at the middle portion including the notches to Dₒ₂, and decreasing yet again at the portion of the distal end from which the teeth extend to Dₒ₃. Preferably, the sleeve is comprised of stainless steel or titanium.

The slide 16 is also generally cylindrical and has an inner diameter dᵢ and an outer diameter dₒ. Outer diameter dₒ is slightly smaller than the inner diameter Dᵢ of the sleeve 14. Thus, the slide is axially disposed with the sleeve 14 as shown in FIGS. 1, 4 and 5. The slide 16 includes a first end 30, a middle portion 31 and a second end 32. The slide 16 defines a spring arm 34 formed via slots 36 extending from about the middle portion 31 towards the second end 32. Thus, the arm has a fixed end 35 in the middle portion 31 and a free end 37 at the second end 32. Since the arm 34 acts like a spring, it may be deflected toward a center 39 of the slide. An edge 38 extends radially from the arm 34 at its free end 37. The spring arm 34 further includes a tab 40 positioned between the fixed end 35 and the free end 37. The tab 40 includes a stem 42 and a pressing member 44 defining ridges 46. Preferably, the slide is comprised of stainless steel or titanium. Alternatively, the slide may be formed of injection molded plastic.

The slide 16 is positioned axially within the sleeve 16 with its second end 32 pointing towards the distal end 22 of the sleeve 14. The channel 26 of the sleeve 14 receives the stem 42 of the tab 40 so that the edge 38 of the slide 16 cooperates with the pairs of notches 28 of the sleeve 14 and the channel. Thus, the edge 38 may be positioned into any one of the pairs of notches 28. Since the arm 34 is a spring, the edge 38 locks into the selected pair of notches. Thus, when the edge 38 is locked into a pair of notches 28, the slide 16 is stabilized in position and cannot be axially displaced within the sleeve. Applying a slight force to the tab 40 against the spring arm 34 causes the arm to deflect inwardly toward center 39, thereby unlocking the edge 38 from the pair of notches. The slide 16 may then be axially moved to a different position. Thus, the desired depth of the hole may be set by deflecting the arm, axially displacing the slide to line the edge up with the proper pair of notches and releasing the arm from deflection to allow the edge to lock into the pair of notches corresponding to the desired depth.

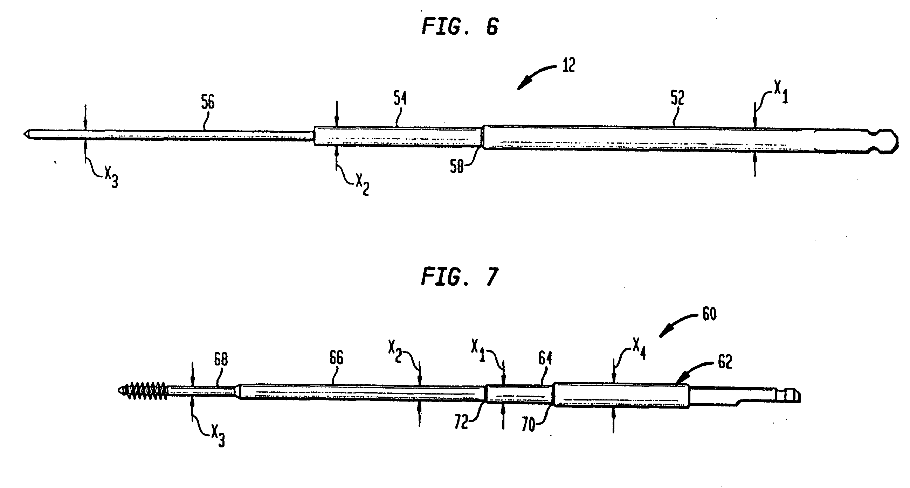

The drill shaft 12 for use with the drill guide 10 according to the present invention is illustrated in FIG. 6. The drill shaft defines a first end 52, a middle portion 54 and a second end or tip 56 and is stepped in diameter. Thus, the diameter of the drill shaft 50 at its first end 52 is X₁, decreasing to X₂ at its middle portion 54 and terminating at X₃ at tip 56. The change in diameter between the first end 52 and middle portion 54 forms shoulder 58. The diameter X₂ of middle portion 54 is slightly smaller than the inner diameter dᵢ of the slide 16. Thus, the drill shaft 12 may be axially inserted into the slide 16, which is positioned within the sleeve 14 as shown in FIG. 5. Since the middle portion 54 of the drill shaft 12 fits within the slide 16, the middle portion 54 prevents the arm 34 of the slide from deflecting inwardly toward the center 39 and thus, unlocking. Thus, once within the sleeve 16, the middle portion 54 assists in locking the edge 38 of the arm 34 into position within the pair of opposite notches. The diameter X₂ of the middle portion also has enough clearance to allow for rotation and movement of the drill within the slide. The shoulder 58 of the drill shaft abuts against the first end 30 of the slide 16 as drilling occurs to stop further advancement of the drill. Therefore, the shape of the drill shaft acts as a constraint to gauge the depth of the hole to be drilled. In one embodiment, the drill guide 10 is capable of controlling the drilling of a hole to a depth in the range of about 6mm to about 54mm. Such a range of depth is appropriate and preferable for the cervical spine.

Referring now to FIG. 7, a tap 60 may also be used with the guide 10 to tap a hole that has been drilled in the bone. Thus, the guide 10 also regulates the depth at which the tapping is complete. The tap 60 includes a first end 62, a first middle portion 64, a second middle portion 66, and a second end 68. The tap 60 is also stepped in diameter. The first end 62 has the largest diameter of X₄. The first middle portion has a diameter X₁ which is substantially equal to the diameter of the first end 52 of the drill shaft 12, but less than the diameter X₄ of the first end 62. A first shoulder 70 is formed at the junction of the first end 62 and the first middle portion 64. The second middle portion 66 has a diameter X₂, which is substantially equal to the diameter of the middle portion 54 of the drill shaft 12, and smaller than the diameter X₁ of the first middle portion 64. A second shoulder 72 is formed at the junction of the first middle portion 64 and the second middle portion 66. Finally, the second end has a diameter X₃ which is substantially equal to the diameter X₃ of the second end 56 of the drill shaft 12, and smaller than the diameter X₂ of the second middle portion 66.

Since the diameter X₂ of the second middle portion 66 of the tap 60 is slightly smaller than the inner diameter dᵢ of the slide 16, the tap 60 may be inserted into the slide 16 at the second middle portion 66. The diameter X₁ of the first middle portion is larger than the inner diameter dᵢ of the slide 16. Therefore, the second middle portion should abut the slide to prevent the tap from advancing. It is the first shoulder 70, however that acts as the constraint. Specifically, the diameter X₄ of the first end 62 is larger than the outside diameter Dₒ₁ of the sleeve. Thus, the tap is constrained from tapping more deeply when the first shoulder 70 abuts the sleeve 14.

Preferably, when used for the cervical spine, the tap 60 is capable of tapping to a depth in the range of about 6mm to about 24mm. Thus, the first shoulder 70 is positioned such that it abuts the sleeve 14 when the tap reaches a depth of 24 mm. This is an added safety component, since tapping the bone too deeply could cause severe damage.

The handle 18 is attached to the sleeve 14 at its proximal end 20. The handle extends at an angle such that the guide 10 may be comfortably handled by a surgeon. The handle 18 includes a grip 48.

In operation, a surgeon performing an orthopedic procedure requiring the drilling and/or tapping of a hole in a bone to a specific depth would first prepare the surface of the bone, which is already exposed. The surgeon, preferably, marks the location of the hole using, for example, an awl to create a dimple in the bone.

Next, the surgeon holds the guide 10 via the grip 48 in one hand. The guide is then set to control the desired depth of the hole to be drilled into the bone. The depth of the hole may be set, for example, within the range of about 6mm to 54mm, which is an appropriate range for the cervical spine. Preferably, each pair of opposite notches is marked with the depth of the hole to which the pair corresponds. In order to set the depth, the slide must be moved. Thus, the tab 40 is pressed to deflect the arm 34 inwardly, thereby unlocking the edge 38 from the notches 28. Having unlocked the slide 16, it may easily be moved axially within the sleeve by the surgeon using the thumb of the hand while gripping the guide 10. The ridges 46 on the pressing member 44 prevent the thumb from slipping off the tab and facilitate movement of the slide 16. The edge 38 must be lined up with the pair of notches that corresponds to the desired depth of the hole. The tab may then be released to lock the slide into position.

Once the desired depth of the hole has been set on the guide 10, the teeth 24 are pressed onto the bone so that the dimple is centered within the teeth 24. The teeth 24 ensure that the drill guide does not slip from its placement on the bone. Using the free hand, the surgeon then inserts the drill shaft 12 through the sleeve and slide until the tip 56 contacts the surface of the bone at the dimple. The surgeon then manually turns the drill using his or her free hand, which advances the drill shaft 12 axially within the slide 16 to drill the hole in the bone. As the hole is drilled, the shaft advances until the shoulder 58 contacts or abuts the first end 30 of the slide 16. Since the diameter X₁ of the first end 52 of the drill shaft 12 is larger than the inside diameter dᵢ of the slide 16, the drill cannot advance further. Thus, the hole has reached the desired depth, and the surgeon can stop drilling and remove the drill shaft from the drill guide 10.

Of course, the present invention also contemplates inserting the drill shaft into the sleeve prior to the guide 10 contacting the bone.

If desired, the hole then may be tapped using tap 60. Similar to using the drill, the depth of the desired tapping must first be set on the guide 10 by the surgeon. The depth of the tapping may be set within a range of about 6mm to about 24mm. Thus, at 24 mm, the first shoulder 70 will abut the sleeve 14. The surgeon sets the depth of the tapping on the guide by pressing the tab 40 to deflect the spring arm 34 inwardly. The slide may then be moved axially within the sleeve to line the edge 38 up with the notches 28 that correspond to the desired depth of the tapping. Preferably, the depth of the tapping may be set to within a range of about 6mm to about 24 mm. Once the edge is lined up with the pair of notches that correspond to the desired depth of tapping, the tab may be released so that the springing motion of the arm allows the tab to lock into position. The slide 16 is then locked into position.

Next, the surgeon inserts the tap 60 into the slide until the second end 68 of the tap is approximately in line with the teeth 24 of the guide. The guide can then be positioned onto the bone using the teeth. The teeth are placed onto the bone, surrounding the hole to be tapped. The tap is then operated so that it advances axially within the guide to tap the hole. Thus, the tap advances until the second shoulder 72 abuts the slide 16. In the alternative, if the depth of desired tapping is 24mm, then the tap advances until the first shoulder 70 abuts the proximal end 20 of the sleeve 14. This is an added safety feature to ensure that the tapping does not occur past this point.

Of course, the drill guide of the present invention may be used for drilling holes for all types of bone screws and not just pedicle screws. For example, the drill guide may be used for trauma applications and other orthopedic applications.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. An instrument for use in drilling a hole in a bone, comprising:
a sleeve (14), including a depth gauge for setting the depth of the hole to be drilled into the bone, said sleeve including an end for contacting the bone;
a slide (16), including a releasable locking mechanism cooperable with said depth gauge, said slide being axially disposed within said sleeve; and
a drill including a drill shaft (12) defining a shoulder (58), wherein said shaft is axially received within said slide and advances to drill the hole in the bone until said shoulder contacts said slide, wherein said releasable locking mechanism of said slide includes a spring arm (34) deflectable towards a center of said slide for unlocking said slide from said depth gauge of said sleeve

2. An instrument according to claim 1, wherein said depth gauge comprises a channel (26) formed in said sleeve, said channel defining a plurality of receiving members (28), and said spring arm having an edge (38) extending radially therefrom for locking into said receiving members, wherein deflecting said arm towards a center of said slide unlocks said edge from said receiving members.

3. An instrument according to claim 2, wherein said arm further includes a tab (40) extending radially from said arm and being aligned within said channel, wherein applying a force to said tab deflects said arm.

4. An instrument according to claim 2, wherein each said receiving member is labeled with the corresponding depth.

5. An instrument according to claim 2, wherein each said receiving member comprises a form selected from the group consisting of notches, holes and indentations.

6. An instrument according to claim 1, wherein said sleeve further includes teeth (24) extending axially therefrom for contacting the bone.

7. An instrument according to claim 1, wherein said drill defines a first end, a middle portion having a diameter smaller than an inner diameter of said slide, and a second end, wherein said shoulder is formed between said first end and said middle portion and said middle portion is axially disposed within said slide.

8. An instrument according to claim 1, wherein said sleeve is comprised of material selected from the group consisting of stainless steel and titanium.

9. An instrument according to claim 1, wherein said slide is comprised of material selected from the group consisting of stainless steel, titanium, and plastic.

10. An instrument according to claim 1, wherein said depth gauge is adaptable to control the depth of the hole in the range of about 6mm to about 54mm.

11. An instrument according to claim 10, wherein said depth gauge is adaptable to control the depth of the hole from about 10mm to about 14mm.

12. An instrument according to claim 1, further comprising a tap (60) including a tap shaft defining a first shoulder, wherein said tap is axially received within said slide and advances to tap the hole in the bone until said first shoulder of said tap contacts said slide.

13. An instrument according to claim 12, wherein said depth gauge is adaptable to control the depth of tapping in the range of about 6mm to about 24mm.

14. An instrument according to claim 12, wherein said tap further defines an end having an end diameter, a first middle portion having a first middle diameter smaller than said end diameter , and a second middle portion having a second middle diameter smaller than said first middle portion diameter, wherein said first shoulder is formed between said first middle portion and said second middle portion and a second shoulder is formed between said end and said first middle portion.

15. An instrument according to claim 14, wherein said end diameter is larger than an inner diameter of said sleeve such that said second shoulder abuts said sleeve when the hole is tapped to a predetermined maximum depth.

16. An instrument according to one of claims 2 to 15, wherein said receiving members are pairs of notches, each said pair of notches corresponding to a depth of the hole to be drilled into the bone.

17. An instrument according to one of claims 1 to 16,
wherein said slide is adapted to receive one or both of a drill and a tap, each of said drill and said tap including a shaft defining a shoulder for contacting said slide to stop advancement of said drill or said tap.

## Patentansprüche

1. Instrument zur Verwendung beim Bohren eines Lochs in einen Knochen, umfassend:
eine Hülse (14), die eine Tiefenjustierung zur Einstellung der Tiefe des in den Knochen zu bohrenden Lochs umfaßt, wobei die Hülse ein Ende zum Kontaktieren des Knochens umfaßt;
einen Schieber, der einen lösbaren Verriegelungsmechanismus umfaßt, der mit der Tiefenjustierung (16) zusammenwirken kann, wobei der Schieber axial in der Hülse angeordnet ist; und
einen Bohrer, der einen Bohrschaft (12) umfaßt, der einen Anschlag (58) definiert, wobei der Schaft axial im Schieber aufgenommen ist und sich vorwärts bewegt, um das Loch in den Knochen zu bohren, bis der Anschlag den Schieber kontaktiert, wobei der lösbare Verriegelungsmechanismus des Schiebers einen Federarm (34) umfaßt, der zu einer Mitte des Schiebers zum Entriegeln des Schiebers von der Tiefenjustierung der Hülse ablenkbar ist.

2. Instrument nach Anspruch 1, bei dem die Tiefenjustierung einen in der Hülse gebildeten Kanal (26) umfaßt, wobei der Kanal eine Mehrzahl an Aufnahmeelementen (28) definiert, und der Federarm eine Kante (38) aufweist, die sich davon radial zum Verriegeln in die Aufnahmeelemente erstreckt, bei dem ein Ablenken des Arms zu einer Mitte des Schiebers die Kante vom Aufnahmeelement entriegelt.

3. Instrument nach Anspruch 2, bei dem der Arm ferner einen Nase (40) umfaßt, die sich radial ausgehend vom Arm erstreckt und mit dem Kanal ausgerichtet ist, bei dem ein Anwenden einer Kraft auf die Nase den Arm ablenkt.

4. Instrument nach Anspruch 2, bei dem jedes der Aufnahmeelemente mit der entsprechenden Tiefe beschriftet ist.

5. Instrument nach Anspruch 2, bei dem jedes Aufnahmeelemente eine aus der Gruppe ausgewählte Form aufweist, die aus Kerben, Löchern und Vertiefungen besteht.

6. Instrument nach Anspruch 1, bei dem die Hülse ferner Zähne (24) umfaßt, die sich axial davon zum Kontaktieren des Knochens erstrecken.

7. Instrument nach Anspruch 1, bei dem der Bohrer ein erstes Ende, einen Mittenabschnitt mit einem Durchmesser kleiner als ein Innendurchmesser des Schiebers, und ein zweites Ende definiert, bei dem der Anschlag zwischen dem ersten Ende und dem Mittenabschnitt gebildet ist und der Mittenabschnitt axial im Schieber angeordnet ist.

8. Instrument nach Anspruch 1, bei dem die Hülse ein aus der Gruppe ausgewähltes Material umfaßt, die aus rostfreiem Stahl und Titan besteht.

9. Instrument nach Anspruch 1, bei dem der Schieber ein aus der Gruppe ausgewähltes Material umfaßt, die aus rostfreiem Stahl, Titan und Kunststoff besteht.

10. Instrument nach Anspruch 1, bei dem die Tiefenjustierung zum Einstellen der Tiefe des Lochs in einem Bereich von etwa 6 mm bis etwa 54 mm einstellbar ist.

11. Instrument nach Anspruch 10, bei dem die Tiefenjustierung zum Einstellen der Tiefe des Lochs von etwa 10 mm bis etwa 14 mm einstellbar ist.

12. Instrument nach Anspruch 1, das ferner einen Gewindebohrer (60) umfaßt, der einen Gewindebohrerschaft umfaßt, der einen ersten Anschlag definiert, wobei der Gewindebohrer axial in dem Schieber aufgenommen ist und sich vorwärts bewegt, um ein Gewinde in das Loch in den Knochen zu schneiden, bis der erste Anschlag des Gewindebohrers den Schieber kontaktiert.

13. Instrument nach Anspruch 12, bei dem die Tiefenjustierung zum Einstellen der Tiefe der Gewindebohrung in dem Bereich von etwa 6 mm bis etwa 24 mm einstellbar ist.

14. Instrument nach Anspruch 12, bei dem der Gewindebohrer ferner ein Ende mit einem Endendurchmesser, einen ersten Mittenabschnitt mit einem ersten Mittendurchmesser kleiner als der Endendurchmesser, und einen zweiten Mittenabschnitt mit einem zweiten Mittendurchmesser kleiner als der erste Mittenabschnittdurchmesser definiert, bei dem der erste Anschlag zwischen dem ersten Mittenabschnitt und dem zweiten Mittenabschnitt gebildet ist und ein zweiter Anschlag zwischen dem Ende und dem ersten Mittenabschnitt gebildet ist.

15. Instrument nach Anspruch 14, bei dem der Endendurchmesser größer als ein Innendurchmesser der Hülse ist, so daß der zweite Anschlag an die Hülse anstößt, wenn in das Loch ein Gewinde bis zu einer vorbestimmten Maximaltiefe geschnitten wird.

16. Instrument nach einem der Ansprüche 2 bis 15, bei dem die Aufnahmeelemente Paare von Kerben sind, wobei jedes Paar von Kerben einer Tiefe des in den Knochen zu bohrenden Lochs entspricht.

17. Instrument nach einem der Ansprüche 1 bis 16, bei dem der Schieber ausgelegt ist, einen oder beide eines Bohrers und eines Gewindebohrers aufzunehmen, wobei sowohl der Bohrer als auch der Gewindebohrer einen Schaft umfassen, der einen einen Anschlag definierenden Schaft zum Kontaktieren des Schiebers umfaßt, um ein Vorwärtsbewegen des Bohrers oder des Gewindebohrers zu stoppen.

## Revendications

1. Instrument destiné à être utilisé pour percer un trou dans un os, comprenant :
un manchon (14) comportant un indicateur d'enfoncement pour régler la profondeur du trou à percer dans l'os, ledit manchon comprenant une extrémité destinée à venir en contact avec l'os ;
une coulisse (16), comprenant un mécanisme de blocage amovible pouvant coopérer avec ledit indicateur d'enfoncement, ladite coulisse étant disposée axialement dans ledit manchon ; et
un foret comprenant une tige de foret (12) définissant un épaulement (58), dans lequel ladite tige est reçue axialement à l'intérieur de ladite coulisse et se déplace vers l'avant pour percer le trou dans l'os jusqu'à ce que ledit épaulement vienne en contact avec ladite coulisse, ledit mécanisme de blocage amovible de ladite coulisse comprenant un bras de ressort (34) pouvant fléchir vers un centre de ladite coulisse afin de débloquer ladite coulisse dudit indicateur d'enfoncement dudit manchon.

2. Instrument selon la revendication 1, dans lequel ledit indicateur d'enfoncement comprend un canal (26) formé dans ledit manchon (28), et ledit bras de ressort présente un bord (38) s'étendant radialement depuis celui-ci destiné à se bloquer dans lesdits éléments de réception, le fléchissement dudit bras vers un Centre de ladite coulisse débloquant ledit bord desdits éléments de réception.

3. Instrument selon la revendication 2, dans lequel ledit bras comprend en outre une languette (40) s'étendant radialement depuis ledit bras et alignée dans ledit canal, le fait d'appliquer une force sur ladite languette entraînant le fléchissement dudit bras.

4. Instrument selon la revendication 2, dans lequel la profondeur correspondante est indiquée sur chaque élément de réception.

5. Instrument selon la revendication 2, dans lequel chaque élément de réception comprend une forme choisie dans le groupe constitué d'encoches, de trous et d'indentations.

6. Instrument selon la revendication 1, dans lequel ledit manchon comprend en outre des dents (24) s'étendant axialement depuis ce dernier pour venir en contact avec l'os.

7. Instrument selon la revendication 1, dans lequel ledit foret définit une première extrémité, une partie centrale ayant un diamètre inférieur à un diamètre interne de ladite coulisse, et une seconde extrémité, ledit épaulement étant formé entre ladite première extrémité et ladite partie centrale et ladite partie centrale étant disposée axialement dans ladite coulisse.

8. Instrument selon la revendication 1, dans lequel ledit manchon comprend un matériau sélectionné dans le groupe constitué d'acier inoxydable et de titane.

9. Instrument selon la revendication 1, dans lequel ladite coulisse comprend un matériau sélectionné dans le groupe constitué d'acier inoxydable, de titane et de plastique.

10. Instrument selon la revendication 1, dans lequel ledit indicateur d'enfoncement peut être adapté pour contrôler la profondeur du trou dans la gamme d'environ 6 mm à environ 54 mm.

11. Instrument selon la revendication 10, dans lequel ledit indicateur d'enfoncement peut être adapté pour contrôler la profondeur du trou d'environ 10 mm à 14 mm.

12. Instrument selon la revendication 1, comprenant en outre un taraud (60) avec une tige de taraud définissant un premier épaulement, dans lequel ledit taraud est reçu axialement dans ladite coulisse et se déplace vers l'avant pour tarauder le trou dans l'os jusqu'à ce que ledit premier épaulement dudit taraud vienne en contact avec ladite coulisse.

13. Instrument selon la revendication 12, dans lequel ledit indicateur d'enfoncement peut être adapté pour contrôler la profondeur de taraudage dans la gamme d'environ 6 mm à environ 24 mm.

14. Instrument selon la revendication 12, dans lequel ledit taraud définit en outre une extrémité ayant un diamètre d'extrémité, une première partie centrale ayant un premier diamètre central inférieur audit diamètre d'extrémité, et une seconde partie centrale ayant un second diamètre central inférieur au diamètre de la première partie centrale, ledit premier épaulement étant formé entre ladite première partie centrale et ladite seconde partie centrale et un second épaulement étant formé entre ladite extrémité et ladite première partie centrale.

15. Instrument selon la revendication 14, dans lequel ledit diamètre d'extrémité est supérieur à un diamètre interne dudit manchon, de telle sorte que ledit second épaulement vienne en butée contre ledit manchon lorsque le trou est taraudé jusqu'à une profondeur maximale prédéterminée.

16. Instrument selon l'une quelconque des revendications 2 à 15, dans lequel lesdits éléments de réception sont des paires d'encoches, chacune desdites paire d'encoches correspondant à une profondeur du trou à percer dans l'os.

17. Instrument selon l'une quelconque des revendications 1 à 16, dans lequel ladite coulisse est adaptée pour recevoir un foret et un taraud ou bien l'un des deux, chacun d'entre eux comprenant une tige définissant un épaulement destiné à venir en contact avec ladite coulisse pour arrêter la progression vers l'avant dudit foret ou dudit taraud.
